(19) Europäisches Patentamt
European Patent Office
Office européen des brevets

(11) Publication number: 0 255 097 A2

# (12) EUROPEAN PATENT APPLICATION

(21) Application number: 87110871.8

(22) Date of filing: 27.07.87

(51) Int. Cl.[3]: C 08 K 5/52
C 08 L 23/02, A 61 L 31/00

(30) Priority: 28.07.86 JP 177324/86

(43) Date of publication of application: 03.02.88 Bulletin 88/5

(84) Designated Contracting States: BE CH DE FR GB LI NL

(71) Applicant: ADEKA ARGUS CHEMICAL CO., Ltd.
5-2-13 Shirahata, Urawa City
Saitama Prefecture(JP)

(72) Inventor: Tajima, Kenji
1200-325 Nishibessho,
Kuwana, Mie(JP)

(72) Inventor: Tsuboi, Tetsuo
244-4, 1443-80 Ishizaka,
Hatoyama, Saitama,(JP)

(72) Inventor: Takeuchi, Takashi
2-17-4 Shirahata,
Urawa, Saitama(JP)

(74) Representative: Weinhold, Peter, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

## (54) Process for improving resistance to deterioration when exposed to sterilizing radiation of polyolefin resins.

(57) A process for improving resistance to deterioration when exposed to sterilizing radiation of polyolefin resins which comprises combining with polyolefin resin an amount sufficient to improve resistance of the polyolefin resin to such deterioration of a phosphite having the formula:

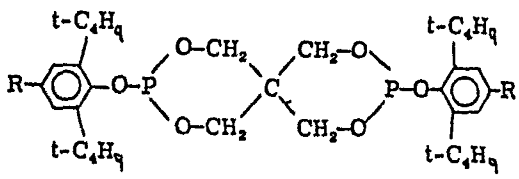

(I)

wherein R is alkyl having from one to about nine carbon atoms; and then exposing the polyolefin resin to sterilizing radiation.

EP 0 255 097 A2

## SPECIFICATION

Polyolefins such as polyethylene and polypropylene formed into medical instruments such as injectors, pincettes and clamps or food wrapping materials by molding or extrusion are widely used in medical and food apparatus because of their favorable mechanical physical and inert chemical properties. In such uses, the polyolefin is often exposed to sterilizing radiation. However, such exposure severely deteriorates the polymer properties, resulting in cracking, lowered mechanical strength, and discoloration. This deterioration is a result of degradation of the polymer molecule. Stabilizers such as phenolic antioxidants, organic phosphorus compounds and hindered amine-type light stabilizers have accordingly been used, either singly or in combinations.

Among the proposed stabilizer systems are symmetrical triarylphosphites (Japanese Patent Laid-Open No. 179234/1982) blends of hindered amine compounds and phosphite esters (Japanese Patent Laid-Open No. 49737/1983) and combinations of hindered amine compound and bis(2, 4-di-tert-butylphenyl) pentaerythritoldiphosphite or bis(2-tert-butyl-4-methylphenyl) pentaerythritoldiphosphite (Japanese Patent Laid-Open No. 118447/1986). The effects of these proposed systems leave much to be desired, and better stabilizers are therefore needed.

Cold sterilization is a widely used sterilization process in which the article or material to be sterilized is subjected to $\gamma$-radiation in apparatus capable of generating the requisite $\gamma$-rays in sufficient amount. Such apparatus is conventional, and forms no part of this invention. Such sterilizers are available commercially, especially adapted for sterilizing medical instruments, wrapped articles, such as foodstuffs, etc., of which polyolefin resin is a component. The term "sterilizing radiation" as used herein refers to $\gamma$-ray radiation in such apparatus, as well as any other available source of $\gamma$-radiation, and the phosphites of Formula I are effective in inhibiting the deteriorating effects of $\gamma$-radiation in polyolefin resins.

Phosphites have long been employed in conjunction with other heat stabilizers such as polyhydric phenols, phenolic antioxidants, polyvalent metal salts of fatty acids, and thioethers in the stabilization of polypropylene and other polyolefins against degradation upon heating or ageing under atmospheric conditions. The polyhydric phenol is thought to function as an antioxidant in such combinations. Such phosphite stabilizers normally contain alkyl or aryl radicals in sufficient number to satisfy the three valences of the phosphite, and may be combined with phenol in the stabilizer system. Typical phosphites are described in the patent literature, for example, Hecker et al, U.S. Patents Nos.

3,244,650 of April 5, 1966 and 3,255,136 of June 7, 1966, and Kauder et al Nos. 3,655,832 of April 11, 1972 and 3,662,032 of May 9, 1972.

The importance of organic phosphites as stabilizers for polyolefins has led to the development of a large variety of special phosphites intended to provide improved stabilizing effectiveness and compatibility and ease of compounding with the resin and with other stabilizers commonly used.

Among these special phosphites, L. Friedman, U.S. Patent No. 3,047,608 of July 31, 1962 discloses a class of spiro-biphosphites having the formula:

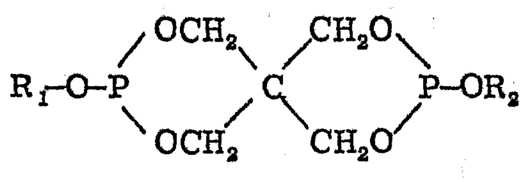

in which $R_1$ and $R_2$ are alkyl or aryl.

Hechenbleikner, U.S. Patent No. 4,290,976, patented September 22, 1981, states that dialkyl pentaerythritol diphosphites having the structural formula

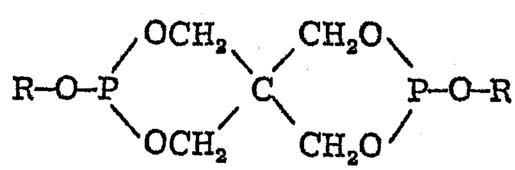

where R and R are alkyl groups have been known for some time as effective stabilizers for polyolefins.

Fisch, Blum and Brecker EPO patent Publication number 0 143 464, date of publication June 5, 1985,

Bulletin 85/23 discloses pentaerythritol-spiro-bis-phosphite compositions having an improved hydrolytic stability, comprising

(1) a pentaerythritol-spiro-bis-phosphite of the formula:

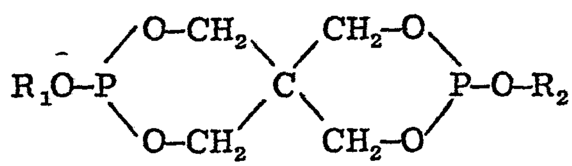

wherein:

$R_1$ and $R_2$ are selected from the group consisting of alkyl and alkylaryl groups having at least fourteen carbon atoms up to about thirty-six carbon atoms; and

(2) a long-chain aliphatic amine in an amount to improve the hydrolytic stability of the phosphite.

Exemplary $R_1$ and $R_2$ alkaryl groups in the phosphite include octylphenyl, 2, 6-di-t-butyl-4-methylphenyl, 2, 6-di-t-butyl-4-methoxycarbonyl, ethylphenyl, isooctylphenyl, t-octylphenyl, nonylphenyl, 2, 4-di-t-butylphenyl, benzylphenyl and phenethylphenyl.

Exemplary pentaerythritol spiro-bis-phosphites include dimyristyl pentaerythritol diphosphite, dihexadecyl pentaerythritol diphosphite, distearyl pentaerythritol diphosphite, bis(2, 4-di-t-butylphenyl) pentaerythritol diphosphite, di-(2, 6-di-t-butylphenyl) pentaerythritol diphosphite, di-(2-t-butyl-4-methylphenyl) pentaerythritol diphosphite, 2, 4-di-t-butyl-6-methylphenyl octylphenyl pentaerythritol diphosphite,

2,4-di-t-butyl-6-methylphenyl nonylphenyl pentaerythritol diphosphite, bis(2,6-di-t-butyl-4-methylphenyl) pentaerythritol diphosphite, bis(2,6-di-t-butyl-4-ethylphenyl) pentaerythritol diphosphite, 2,6-di-t-butyl-4-methylphenyl-2,6-di-t-butylphenyl pentaerythritol diphosphite, 2,6-di-t-butyl-4-methylphenyl-2,4-di-t-butylphenyl pentaerythritol diphosphite, 2,6-di-t-butyl-4-methylphenyl-2,4-di-t-octylphenyl pentaerythritol diphosphite, 2,6-di-t-amyl-4-methylphenyl phenyl pentaerythritol diphosphite, bis(2,6-di-t-amyl-4-methylphenyl) pentaerythritol diphosphite, and bis(2,6-di-t-octyl-4-methylphenyl) pentaerythritol diphosphite.

These phosphites have been proposed as heat stabilizers, but it has not been suggested that phosphites are effective in inhibiting degradation of polyolefins due to exposure to $\gamma$-ray radiation.

In accordance with the present invention, a process is provided for improving the resistance of polyolefin resins to deterioration when exposed to sterilizing radiation which comprises combining with polyolefin resin an amount sufficient to improve resistance of the polyolefin resin to such deterioration of a phosphite having the formula:

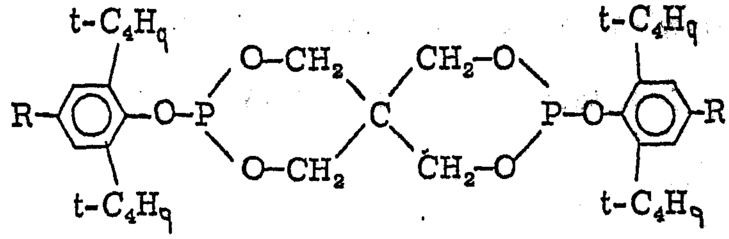 (I)

wherein R is alkyl having from one to about nine carbon atoms; and then exposing the polyolefin resin to sterilizing radiation.

The structure of the pentaerythritol bis-phosphite is important for the stabilizing effect. Phosphites closely related in structure, such as bis(2, 4-di-tert-butylphenyl) pentaerythritol diphosphite, are relatively ineffective, although the only difference is in the ring positions of the tert-butyl group and no methyl or ethyl substituent on the phenyl ring. See the Control, Table I, page 41.

Exemplary R alkyl include, for example, methyl, ethyl, propyl, isopropyl, butyl, secondary-butyl, tertiary-butyl, isobutyl, amyl, tertiary-amyl, isoamyl, hexyl, heptyl, octyl, isooctyl, 2-ethylhexyl, tertiary-octyl, nonyl, isononyl, and tertiary-nonyl.

Specific examples of the phosphites falling within Formula (I) and useful in the practice of the present invention, are bis(2, 6-di-tert-butyl-4-methylphenyl) pentaerythritol diphosphite, bis(2, 6-di-tert-butyl-4-ethylphenyl) pentaerythritol diphosphite, bis(2, 6-di-tert-butyl-4-isopropylphenyl) pentaerythritol diphosphite, bis(2, 4, 6-tri-tert-butylphenyl) pentaerythritol diphosphite, bis(2, 6-di-tert-butyl-4-sec-butylphenyl) pentaerythritol diphosphite, bis(2, 6-di-tert-butyl-4-tert-octylphenyl) pentaerythritol diphosphite and bis(2, 6-di-tert-butyl-4-tert-nonylphenyl) pentaerythritol diphosphite.

The polyolefins whose resistance to deterioration when exposed to sterilizing radiation is improved in accordance with the present invention, include homo- and co-polymers of $\alpha$-olefins, such as ethylene, propylene, butene, pentene, hexene, 4-methylpentene, heptene and octene; random, block or graft copolymers of these $\alpha$-olefins in a major proportion (by weight) with a minor proportion of vinyl ester such as vinyl acetate, unsaturated organic acid (including the salts, amides and amines thereof) such as acrylic acid, maleic anhydride and methyl methacrylate or vinyl silanes such as vinyl trimethoxysilane; as well as such polymers and polymers subjected to chlorination, sulfonation, oxidation, etc.

Specific examples of polyolefins include low, medium and high density polyethylene; polypropylene; polybutene; ethylene-propylene random or block copolymers; ethylene-propylene-butene copolymer; ethylene-butene copolymer; ethylene-4-methylpentene copolymer; propylene-hexene copolymer; and propylene-hexene-butene copolymer.

The phosphites of the invention can be combined with conventional polyolefin resin heat stabilizers, including phenolic antioxidants.

The phenolic antioxidant contains one or more phenolic hydroxyl groups, and one or more phenolic nuclei, and can contain from about eight to about three hundred carbon atoms. In addition, the phenolic nucleus can contain an oxy or thio ether group.

The alkyl-substituted phenols and polynuclear phenols, because of their molecular weight, have a higher boiling point, and therefore are preferred because of their lower volatility. There can be one or a plurality of alkyl groups of one or more carbon atoms. The alkyl group or groups including any alkylene groups between phenol nuclei preferably aggregate at least four carbon atoms. The longer the alkyl or alkylene chain, the better the compatibility with polypropylene, inasmuch as the phenolic compound then acquires more of an aliphatic hydrocarbon character, and therefore there is no upper limit on the number of alkyl carbon atoms. Usually, from the standpoint of availability, the compound will not have more than about eighteen carbon atoms in an alkyl, alicyclidene and alkylene group, and a total of not over about fifty carbon atoms. The compounds may have from one to four alkyl radicals per phenol nucleus.

The phenol contains at least one and preferably at least two phenolic hydroxyls, the two or more hydroxyls being in the same ring, if there is only one. In the case of bicyclic phenols, the rings can be linked by thio or oxyether groups, or by alkylene, alicyclidene or arylidene groups.

The monocyclic phenols which can be employed have the structure:

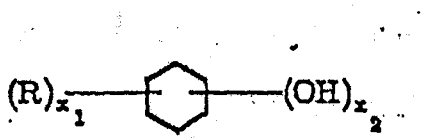

R is selected from the group consisting of hydrogen; halogen; and organic radicals containing from one to about thirty carbon atoms, such as alkyl, aryl, alkenyl, alkaryl, aralkyl, cycloalkenyl, cycloalkyl, alkoxy, and acyl $(R'\underset{O}{\underset{\Vert}{C}}-)$, where R' is aryl, alkyl or cycloalkyl.

$x_1$ and $x_2$ are integers from one to four, and the sum of $x_1$ and $x_2$ does not exceed six.

The polycyclic phenol phenol is one having at least two aromatic nuclei linked by a polyvalent linking radical, as defined by the formula:

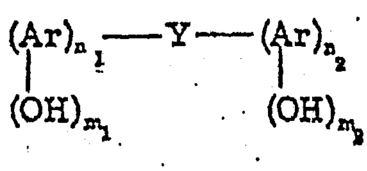

wherein

Y is a polyvalent linking group selected from the group consisting of oxygen; carbonyl; sulfur; sulfinyl; aromatic,

aliphatic and cycloaliphatic hydrocarbon groups; and oxyhydrocarbon, thiohydrocarbon and heterocyclic groups. The linking group can have from one up to twenty carbon atoms.

Ar is a phenolic nucleus which can be a phenyl or a polycarbocyclic group having condensed or separate phenyl rings; each Ar group contains at least one free phenolic hydroxyl group up to a total of five. The Ar rings can also include additional rings connected by additional linking nuclei of the type Y, for example, Ar-Y-Ar-Y-Ar.

$m_1$ and $m_2$ are numbers from one to five, and $n_1$ and $n_2$ are numbers of one or greater, and preferably from one to four.

The aromatic nucleus Ar can, in addition to phenolic hydroxyl groups, include one or more inert substituents. Examples of such inert substituents include hydrogen, halogen atoms, e.g., chlorine, bromine and fluorine; organic radicals containing from one to about thirty carbon atoms, such as alkyl, aryl, alkaryl, aralkyl, cycloalkenyl, cycloalkyl, alkoxy, aryloxy and acyloxy ($R'\underset{\underset{O}{\|}}{C}-O$) where R' is aryl, alkyl or cycloalkyl, or thiohydrocarbon groups having from one to about thirty carbon atoms, and carboxyl ($-\underset{\underset{O}{\|}}{C}-O-$) groups. Usually, however, each aromatic nucleus will not have more than about eighteen carbon atoms in any hydrocarbon substituent group. The Ar group can have from one to four substituent groups per nucleus.

Typical aromatic nuclei include phenyl, naphthyl, phenanthryl, triphenylenyl,anthracenyl, pyrenyl, chrysenyl, and fluoroenyl groups.

When Ar is a benzene nucleus, the polyhydric polycyclic phenol has the structure:

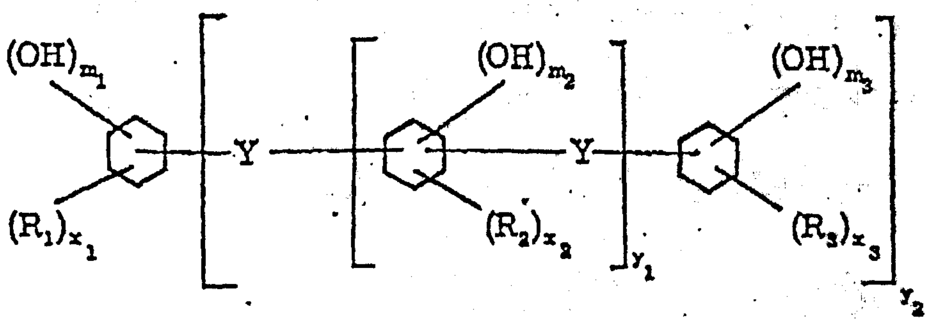

wherein

$R_1$, $R_2$ and $R_3$ are inert substituent groups as described in the previous paragraph;

$m_1$ and $m_3$ are integers from one to a maximum of five;

$m_2$ is an integer from one to a maximum of four;

$x_1$ and $x_3$ are integers from zero to four, and

$x_2$ is an integer from zero to three;

$y_1$ is an integer from zero to about six and

$y_2$ is an integer from one to five, preferably one or two.

Preferably, the hydroxyl groups are located ortho and/or para to Y.

Exemplary Y groups are alkylene, alkylidene, and alkenylene; arylene, alkyl arylene, arylalkylene; cycloalkylene, cycloalkylidene; and oxa- and thia-substituted such groups;

tetrahydrofuranes, esters and triazino groups. The Y groups are usually bi, tri, or tetravalent, connecting two, three or four Ar groups. However, higher valency Y groups connecting more than four Ar groups, can also be used. According to their constitution, the Y groups can be assigned to subgenera as follows:

1) Y groups where at least one carbon in a chain or cyclic arrangement connect the aromatic groups, such as:

$-CH_2-CH_2-$; $-(CH_2)_5-$; $-CH_2-$ ; 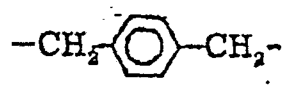 ; 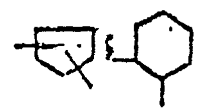 ;

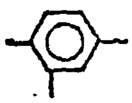 ; 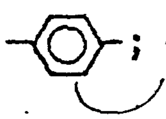 ; 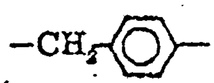 ; 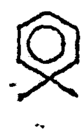 ; 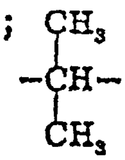 ; 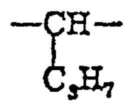 ;

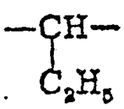 ; 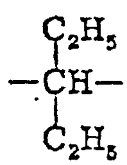 ; 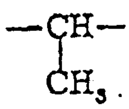 ; 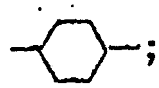 ; 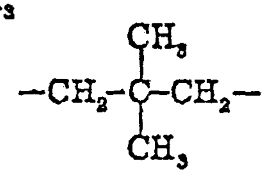 ;

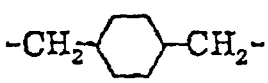 ; 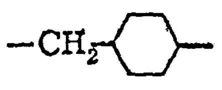 ; 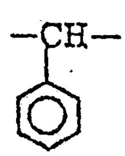 ; 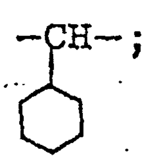 ;

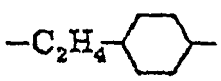 ; 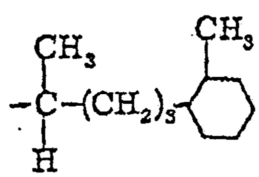 ; 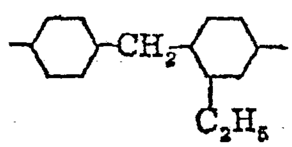

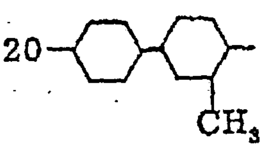 ; 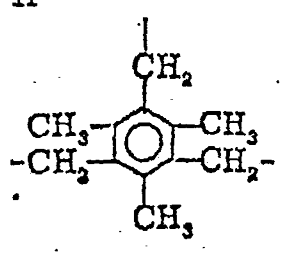 ; 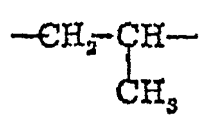 ; 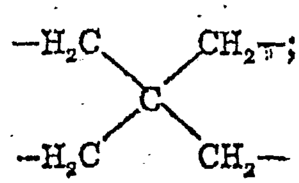 ;

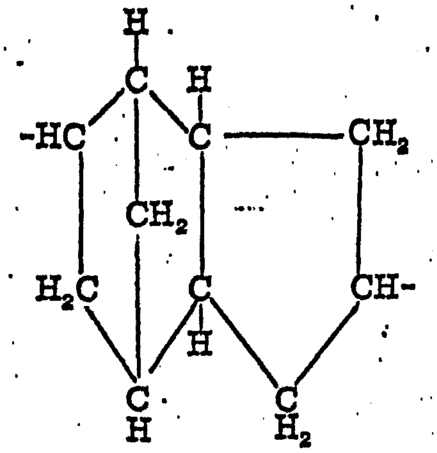 ; 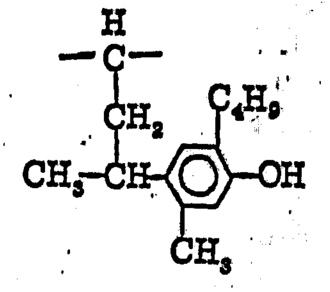 ;

2) Y groups where only atoms other than carbon link the aromatic rings, such as

-O-, -S-, 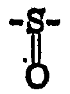 , 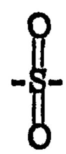 and $-(S)_x-$ where x is a number from one to ten;

3) Y groups made up of more than a single atom including both carbon and other atoms linking the aromatic nuclei, such as:

$-CH_2-O-CH_2-$ ; 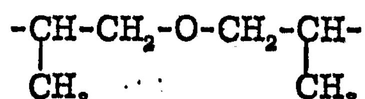 ; $-O-CH_2-CH_2-O-$ ;

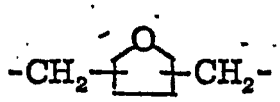 ; 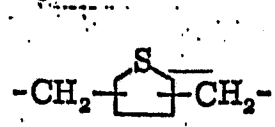 ; $-S-CH_2-S-$;

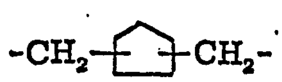 ; 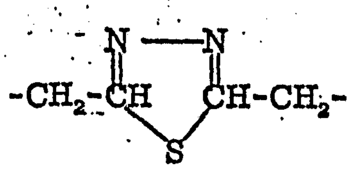 ; 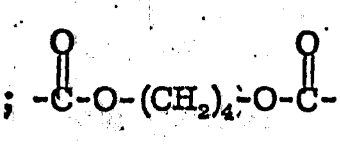 ;

$C[-CH_2OOCCH_2CH_2-]_4\equiv$ ; 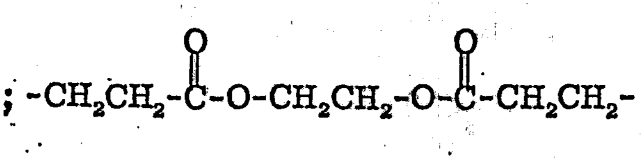 ;

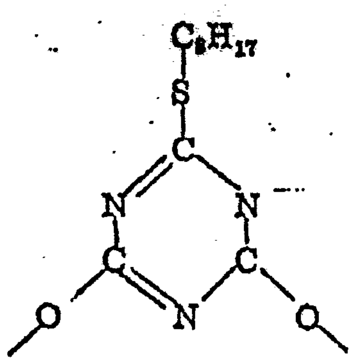 ; $-CH_2-S-$ ; $-CH_2-S-CH_2-$ ; and 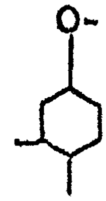

Although the relation of effectiveness to chemical structure is insufficiently understood, many of the most effective phenols have Y groups of subgenus 1), and accordingly this is preferred. Some of these phenols can be prepared by the alkylation of phenols or alkyl phenols with polyunsaturated hydrocarbons such as dicyclopentadiene or butadiene.

Representative phenols include guaiacol, resorcinol monoacetate, vanillin, butyl salicylate, 2,6-di-tert-butyl-4-methyl phenol, 2-tert-butyl-4-methoxy phenol, 2,4-dinonyl phenol, 2,3,4,5-tetradecyl phenol, tetrahydro-α-naphthol, o-, m- and p-cresol, o-, m- and p-phenylphenol, o-, m- and p-xylenols, the carvenols, symmetrical xylenol, thymol, o-, m- and p-nonylphenol, o-, m- and p-dodecyl-phenol, and o-, m- and p-octyl-phenol, o-, and m-tert-butyl-p-hydroxy-anisole, p-n-decyloxy-phenol, p-n-decyloxy-cresol, nonyl-n-decyloxy-cresol, eugenol, isoeugenol, glyceryl monosalicylate, methyl-p-hydroxy-cinnamate, 4-benzyloxy-phenol, p-acetylaminophenol, p-stearyl-aminophenol, methyl-p-hydroxybenzoate, p-dichlorobenzoyl-aminophenol, p-hydroxysalicyl anilide,

stearyl-(3,5-di-methyl-4-hydroxy-benzyl) thioglycolate, stearyl-β-(4-hydroxy-3,5-di-t-butylphenyl) propionate, distearyl-3,5-di-t-butyl-4-hydroxybenzylphosphonate, and distearyl (4-hydroxy-3-methyl-5-t-butyl) benzylmalonate.

Exemplary polyhydric phenols are orcinol, propyl gallate, catechol, resorcinol, 4-octyl-resorcinol, 4-dodecyl-resorcinol, 4-octadecyl-catechol, 4-isooctyl-phloroglucinol, pyrogallol, hexahydroxybenzene, 4-isohexylcatechol, 2,6-di-tertiary-butyl-resorcinol, 2,6-di-isopropyl-phloroglucinol.

Exemplary polyhydric polycyclic phenols are methylene bis-(2,6-di-tertiary-butyl-phenol), 2,2-bis-(4-hydroxy phenyl)-propane, methylene-bis-(p-cresol), 4,4'-benzylidene bis (2-tertiary-butyl-5-methyl-phenol), 4,4'-cyclo-hexylidene bis-(2-tertiary-butylphenol), 2,2'-methylene-bis-(4-methyl-6-(1'-methyl-cyclohexyl)-phenol), 2,6-bis-(2'-hydroxy-3'-tertiary-butyl-5'-methylbenzyl)-4-methylphenol, 4,4'-bis-(2-tertiary-butyl-5-methyl-phenol), 2,2'-bis-(4-hydroxy-phenyl) butane, ethylene bis-(p-cresol), 4,4'-oxobis-phenol, 4,4'-oxobis-(3-methyl-5-isopropyl-phenol), 4,4'-oxobis-(3-methyl-phenol), 2,2'-oxobis-(4-dodecyl-phenol), 2,2'-oxobis-(4-methyl-5-tertiary-butyl-phenol), 4,4'-thio-bis-phenol; 4,4'-thio-bis-(3-methyl-6-tertiary-butyl-phenol), 2,2'-thio-bis-(4-methyl-6-tertiary-butyl-phenol), 4,4'-n-butylidene-(2-t-butyl-5-methyl-

phenol), 2,2'-methylene-bis-(4-methyl-6-(1'-methyl-cyclohexyl)-phenol), 4,4'-cyclohexylene bis-(2-tertiary-butyl-phenol), 2,6-bis-(2'-hydroxy-3'-t-butyl-5'-methyl-benzyl)-4-methyl-phenol, 4,4'-oxobis (naphthalene-1,5-diol), 1,3'-bis-(naphthalene-2,5-diol) propane, and 2,2'-butylene bis-(naphthalene-2,7-diol), (3-methyl-5-tert-butyl-4-hydroxyphenyl)-4'-hydroxy-phenyl) propane, 2,2'-methylene-bis-(4-methyl-5-isopropylphenol), 2,2'-methylene-bis-(4-methyl-5-isopropylphenol), 2,2'-methylene-bis-(5-tert-butyl-4-chlorophenol), (3,5-di-tert-butyl-4-hydroxyphenyl)-(4'-hydroxyphenyl) ethane, (2-hydroxy-phenyl)-(3',5'-di-tert-butyl-4',4-hydroxyphenyl) ethane, 2,2'-methylene-bis-(4-octylphenol), 4,4'-propylene-bis-(2-tert-butyl-phenol), 2,2'-isobutylene-bis-(4-nonylphenol), 2,4-bis-(4-hydroxy-3-t-butyl-phenoxy)-6-(n-octylthio)-1,3,5-triazine, 2,4,6-tris-(4-hydroxy-3-t-butyl-phenoxy)-1,3,5-triazine, 2,2'-bis-(3-t-butyl-4-hydroxyphenyl) thiazolo-(5,4-d) thiazole, 2,2'-bis-(3-methyl-5-t-butyl-4-hydroxyphenyl) thiazolo-(5,4-d)-thiazole, 4,4'-bis-(4-hydroxyphenyl) pentanoic acid octadecyl ester, cyclopentylene-4,4'-bis-phenol, 2-ethylbutylene-4,4'-bisphenol, 4,4'-cyclooctylene-bis-(2-cyclohexylphenol), $\beta,\beta$-thiodiethanol-bis-(3-tert-butyl-4-hydroxyphenoxy acetate), 1,4-butanedio-bis-(3-tert-butyl-4-hydroxyphenoxy acetate), pentaerythritol tetra-(4-hydroxyphenol propionate), 2,4,4'-tri-hydroxy

benzophenone, bis-(2-tert-butyl-3-hydroxy-5-methylphenyl) sulfide, bis-(2-tert-butyl-4-hydroxy-5-methylphenyl) sulfide, bis-(2-tert-butyl-4-hydroxy-5-methylphenyl )sulfoxide, bis-(3-ethyl-5-tert-butyl-4-hydroxybenzyl) sulfide, bis-(2-hydroxy-4-methyl-6-tert-butyl-phenyl) sulfide, 4,4'-bis-(4-hydroxyphenol) pentanoic acid octadecyl thiopropionate ester, 1,1,3-tris-(2'-methyl-4-hydroxy-5'-tert-butylphenyl) butane, 1,1,3-tris-(1-methyl-3-hydroxy-4-tert-butylphenyl) butane, 1,8-bis-(2-hydroxy-5-methylbenzoyl-n-octane, 2,2'-ethylene-bis-[4'-(3-tert-butyl-4-hydroxyphenyl)-thiazole], 1-methyl-3-(3-methyl-5-tert-butyl-4-hydroxybenzyl)-naphthalene, 2,2'-(2-butene)-bis-(4-methoxy-6-tert-butylphenol)-bis-[3,3-bis-(4-hydroxy-3-t-butylphenyl) butyric acid] glycol ester, 4,4'-butylidene-bis-(6-t-butyl-m-cresol), 1,1,3-tris-(2-methyl-4-hydroxy-5-t-butylphenyl) butane, 1,3,5-tris-(3,5-di-t-butyl-4-hydroxybenzyl)-2,4,6-trimethylbenzene, tetrakis [methylene-3 (3,5-di-t-butyl-4-hydroxyphenyl)propionate] methane, 1,3,5-tris-(3,5-di-t-butyl-4-hydroxybenzyl) isocyanurate, 1,3,5-tris-(3,5-di-t-butyl-4-hydroxyphenyl) propionyl-oxyethyl isocyanurate, 2-octylthio-4,6-di-(4-hydroxy-3,5-di-t-butyl) phenoxy-1,3,5-triazine, 4,4'-thiobis-(6-t-butyl-m-cresol) and penta-erythritol hydroxyphenyl propionate.

A particularly desirable class of polyhydric polycyclic phenols are the dicyclopentadiene polyphenols, which are of the type:

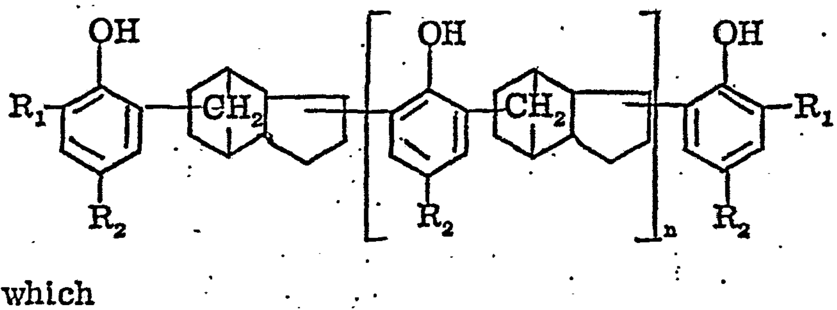

in which

$R_1$ and $R_2$ are lower alkyl, and can be the same or different, and

n is the number of the groups enclosed by the brackets, and is usually from 1 to about 5. These are described in U.S. patent No. 3,567,683, dated March 2, 1971 to Spacht. A commercially available member of this class is Wingstay L, exemplified by dicyclopentadiene tri-(2-tert-butyl-4-methyl-phenol) of the formula:

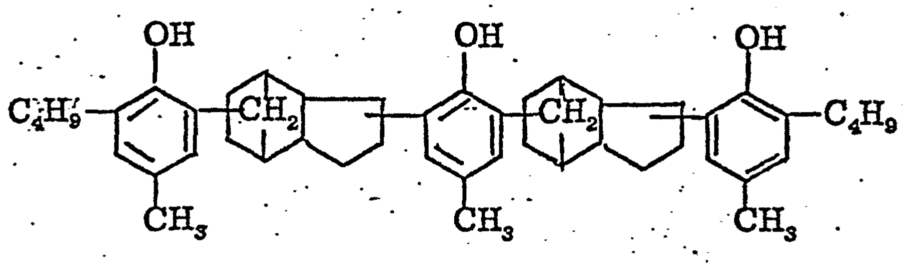

The polyhydric polycyclic phenols used in the invention can also be condensation products of phenols or alkylphenols with hydrocarbons having a bicyclic ring structure and a double bond or two or more double bonds, such as α-pinene, β-pinene,

dipentene, limonene, vinylcyclohexene, dicyclopentadiene, allo-ocimene, isoprene and butadiene. These condensation products are usually obtained under acidic conditions in the form of more or less complex mixtures of monomeric and polymeric compounds. However, it is usually not necessary to isolate the individual constituents. The entire reaction product, merely freed from the acidic condensation catalyst and unchanged starting material, can be used with excellent results. While the exact structure of these phenolic condensation products is uncertain, the Y groups linking the phenolic nuclei all fall into the preferred subgenus 1. For method of preparation, see e.g., U.S. patent No. 3,124,555, U.S. patent No. 3,242,135, and British patent No. 961,504.

In addition, other stabilizers conventionally used as heat and/or light stabilizers for polyolefin resins can be included, such as polyvalent metal salts of organic acids.

When the stabilizer composition is used in conjunction with a polyvalent metal salt of an organic acid, the organic acid will ordinarily have from about six to about twenty-four carbon atoms. The polyvalent metal can be any metal of Group II of the Periodic Table, such as zinc, calcium, cadmium, barium, magnesium and strontium. The alkali metal salts and

heavy metal salts such as lead salts are unsatisfactory. The acid can be any organic non-nitrogenous monocarboxylic acid having from six to twenty-four carbon atoms. The aliphatic, aromatic, alicyclic and oxygen-containing heterocyclic organic acids are operable as a class. By the term "aliphatic acid" is meant any open chain carboxylic acid, substituted, if desired, with nonreactive groups, such as halogen, sulfur and hydroxyl. By the term "alicyclic" it will be understood that there is intended any cyclic acid in which the ring is nonaromatic and composed solely of carbon atoms, and such acids may if desired have inert, nonreactive substituents such as halogen, hydroxyl, alkyl radicals, alkenyl radicals and other carbocyclic ring structures condensed therewith. The oxygen-containing heterocyclic compounds can be aromatic or nonaromatic and can include oxygen and carbon in the ring structure, such as alkyl-substituted furoic acid. The aromatic acids likewise can have nonreactive ring substituents such as halogen, alkyl and alkenyl groups, and other saturated or aromatic rings condensed therewith.

As exemplary of the acids which can be used in the form of their metal salts there can be mentioned the following: hexoic acid, 2-ethylhexoic acid, n-octoic acid, isooctoic acid, capric acid, undecylic acid, lauric acid, myristic acid,

palmitic acid, margaric acid, stearic acid, oleic acid, ricinoleic acid, behenic acid, chlorocaproic acid, hydroxy capric acid, benzoic acid, phenylacetic acid, butyl benzoic acid, ethyl benzoic acid, propyl benzoic acid, hexyl benzoic acid, salicylic acid, naphthoic acid, 1-naphthalene acetic acid, orthobenzoyl benzoic acid, naphthenic acids derived from petroleum, abietic acid, dihydroabietic acid, hexahydrobenzoic acid, and methyl furoic acid.

The water-insoluble salts are preferred, because they are not leached out when the plastic is in contact with water. Where these salts are not known, they are made by the usual types of reactions, such as by mixing the acid, or anhydride with the corresponding oxide or hydroxide of the metal in a liquid solvent, and heating, if necessary, until salt formation is complete.

Also useful are thioether antioxidants. Of these, one class are the thiodipropionic acid esters having the following formula:

$$R_1OOCCH_2CH_2-S-CH_2CH_2COOY$$

in which $R_1$ is an organic radical selected from the group consisting of hydrocarbon radicals such as alkyl, alkenyl, aryl, cycloalkyl and mixed alkyl aryl and mixed alkyl cycloalkyl radicals; hydroxyalkyl and hydroxyalkyloxyalkylene radicals; and esters thereof with aliphatic carboxylic acids; and Y is selected from the group consisting of (a) hydrogen, (b) a second R radical $R_2$, which can be the same as or different from the $R_1$ radical, (c) a polymeric chain of n thiodipropionic acid ester units:

$$-XO[OCCH_2CH_2SCH_2CH_2COOXO]_nOCCH_2CH_2-S-CH_2CH_2COOZ$$

where Z is hydrogen, $R_2$ or M, n is the number of thiodipropionic acid ester units in the chain, and X is a bivalent hydrocarbon group of the type of $R_1$, that is, alkylene, alkenylene, cycloalkylene, mixed alkylene-arylene and mixed alkylene-cycloalkylene radicals; hydroxyalkylene and hydroxyalkyloxyalkylene radicals; and esters thereof with aliphatic carboxylic acids; the value of n can range upwards from 0, but there is no upper limit on n except as is governed by the ratio of carbon atoms to sulfur atoms as stated below; and (d) a polyvalent metal M of Group II of the periodic table such as zinc, calcium, cadmium, barium, magnesium and strontium.

The molecular weights of the R and Y radicals are taken such that with the remainder of the molecule the thiodipropionic ester has a total of from about ten to about sixty carbon atoms per sulfur atom.

Accordingly, the various thiodipropionic acid ester species coming within the above-designated categories within the general formula can be defined as follows:

(a) $R_1OOCCH_2CH_2SCH_2CH_2COOH$

(b) $R_1\ OOCCH_2CH_2SCH_2CH_2COOR_2$

(c) $R_1O[OCCH_2CH_2SCH_2CH_2COOX{-}O]_nOCCH_2CH_2SCH_2CH_2COOZ$

(d) $R_1OOCCH_2CH_2SCH_2CH_2COOM$

In the above formulae $R_1$ and $R_2$, M, X and Z are the same as before and the value of $n_1$ can range upwards from 1, but there is no upper limit on $n_1$ except as is imposed by the ratio of carbon atoms, as stated below. In the polymer (c), as in the other forms of thiodipropionic acid esters, the total number of carbon atoms per sulfur atom is within the range from about ten to about sixty.

The R radical of these esters is important in furnishing compatibility with the polymer. The Y radical is desirably a different radical, $R_2$ or M or a polymer, where R is rather low in molecular weight, so as to compensate for this in obtaining the optimum compatibility and nonvolatility. Where Y is a metal, the thiodipropionic acid ester furnishes the beneficial properties of the polyvalent metal salt which is described above.

The aryl, alkyl, alkenyl, and cycloalkyl groups may, if desired, contain inert, nonreactive substituents such as halogen and other carbocyclic and heterocyclic ring structures condensed therewith.

Typical R radicals are, for example, methyl, ethyl, propyl, isopropyl, butyl, isobutyl, t-butyl, amyl, isoamyl, n-octyl, isooctyl, 2-ethyl hexyl, t-octyl, decyl, dodecyl, octadecyl, allyl, hexenyl, linoleyl, ricinoleyl, oleyl, phenyl,

xylyl, tolyl, ethylphenyl, naphthyl, cyclohexyl, benzyl, cyclopentyl, methylcyclohexyl, ethylcyclohexyl, and naphthenyl, hydroxyethyl, hydroxypropyl, glyceryl, sorbityl, pentaerythrityl, and polyoxyalkylene radicals such as those derived from diethylene glycol, triethylene glycol, polyoxypropylene glycol, polyoxyethylene glycol, and polyoxypropyleneoxyethylene glycol, and esters thereof with any of the organic acids named below in the discussion of the polyvalent-metal salts, including in addition those organic acids having from two to five carbon atoms, such as acetic, propionic, butyric and valeric acids.

Typical X radicals are alkylene radicals such as ethylene, tetramethylene, hexamethylene, decamethylene, alkyl-substituted alkylene radicals such as 1,2-propylene,

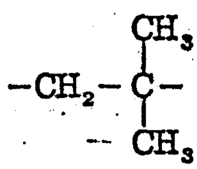 and 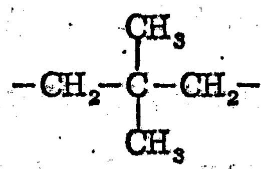

arylene radicals such as phenylene 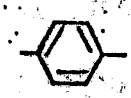

methylenephenylene 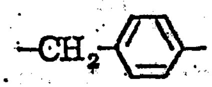

dimethylene phenylene 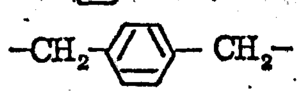

and alicyclylene such as cyclohexylene 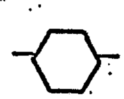

and cyclopentylene 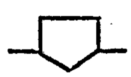

Additional examples of phenolic antioxidants include 2,6-diphenyl-4-octadecyloxyphenol, stearyl (3,5-di-t-butyl-4-hydroxy-phenyl)-propionate, distearyl-3,5-di-t-butyl-4-hydroxybenzyl-phosphonate, thiodiethylenebis(3,5-di-t-butyl-4-hydroxyphenyl-propionate, hexamethylene-bis(3,5-di-t-butyl-4-hydroxyphenyl-propionate, 2-octylthio-4,6-bis(3,5-di-t-butyl-4-hydroxyphenoxy)-s-triazine, 2,2'-methylenebis(4-methyl-6-t-butylphenol), 2,2'-methylenebis(4-ethyl-6-t-butylphenol), bis(3,3-bis(4-hydroxy-3-t-butylphenyl) butyric acid) glycol ester, 2,2'-ethylidenebis(4,6-di-t-butylphenol), 2,2'-ethylidenebis(4-sec-butyl-6-t-butylphenol), 3,6-dioxaoctylenebis(3-methyl-5-t-butyl-4-hydroxyphenylpropionate), bis(2-t-butyl-4-methyl-6-(2-hydroxy-3-t-butyl-5-methyl benzyl) phenyl) terephthalate, 1,3,5-tris(2,6-dimethyl-3-hydroxy-4-t-butylbenzyl) isocyanurate, 1,3,5-tris(3,5-di-t-butyl-4-hydroxybenzyl)-2,4,6-trimethylbenzene, 1,3,5-tris((3,5-di-t-butyl-4-hydroxyphenyl) propionyloxyethyl) isocyanurate, tetrakis (methylene-3-(3,5-di-t-butyl-4-hydroxyphenyl)-propionate) methane.

As exemplary of the thiodipropionic acid esters which can be used, there can be mentioned the following: monolauryl thiodipropionic acid, dilauryl thiodipropionate, butyl stearyl thiodipropionate, 2-ethylhexyl lauryl thiodipropionate, di-2-ethylhexyl-thiodipropionate, diisodecyl thiodipropionate, isodecyl phenyl thiodipropionate, benzyl lauryl thiodipropionate, benzyl phenyl thiodipropionate, the diester of mixed coconut fatty alcohols and thiodipropionic acid, the diester of mixed tallow fatty alcohols and thiodipropionic acid, the acid ester of mixed cottonseed oil fatty alcohols and thiodipropionic acid, the acid ester of mixed soyabean oil fatty alcohols and thiodipropionic acid, cyclohexyl nonyl thiodipropionate, monooleyl thiodipropionic acid, hydroxyethyl lauryl thiodipropionate, monoglyceryl thiodipropionic acid, glyceryl monostearate monothiodipropionate, sorbityl isodecyl thiodipropionate, the polyester of diethylene glycol and thiodipropionic acid, the polyester of triethylene glycol and thiodipropionic acid, the polyester of hexamethylene glycol and thiodipropionic acid, the polyester of pentaerythritol and thiodipropionic acid, the polyester of octamethylene glycol and thiodipropionic acid, the polyester of p-dibenzyl alcohol and thiodipropionic acid, ethylbenzyl lauryl thiodipropionate, strontium stearyl thiodipropionate, magnesium oleyl thiodipropionate, calcium dodecyl-

benzyl thiodipropionate, and mono(dodecylbenzyl) thiodipropionic acid.

These esters are for the most part known compounds, but where they are not available, they are readily prepared by esterification of thiodipropionic acid and the corresponding alcohol.

Also useful are:

(1) Thioalkanoic acid amides of Tokuno et al Japanese patent No. 16,286/68 having the formula:

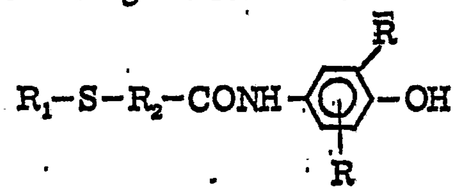

R is alkyl of one to eight carbon atoms, $R_1$ is alkyl of six to twenty-four carbon atoms, and $R_2$ is alkylene of one to six carbon atoms.

(2) Thioalkanoic acid amides of 1,3,5-triazines of Ozeki et al Japanese patent No. 20,366/68 having the formula:

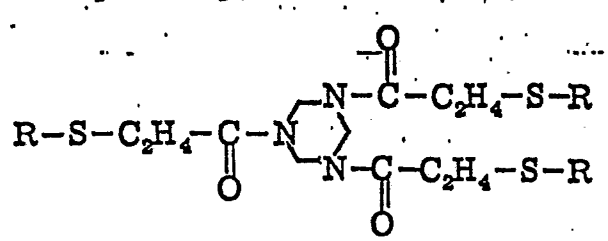

R is alkyl of eight to eighteen carbon atoms.

(3) Bis-thioalkanoic acid amides of Yamamoto et al Japanese patent No. 23,765/68 having the formula:

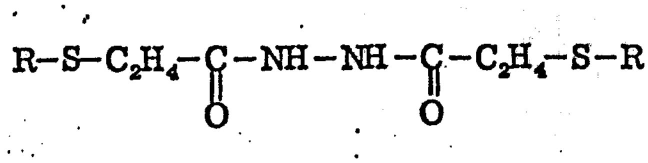

R is alkyl of more than six carbon atoms, aryl or aralkyl.

(4) Bis-thioalkylanoic acid amides of Ozeki et al Japanese patent No. 26,184/69 having the formula:

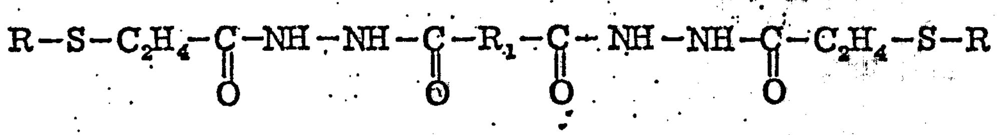

R is alkyl of twelve to eighteen carbon atoms, and $R_1$ is alkylene of one to ten carbon atoms, cycloalkylene, or arylene.

(5) Bis-alkylene thioalkanoic acid amides of Ozeki Japanese patent No. 31,464/69 having the formula:

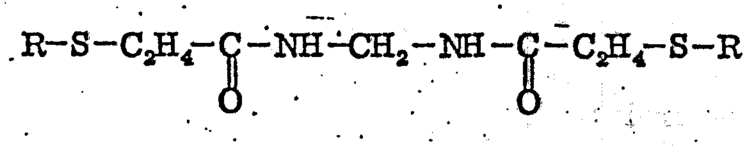

R is alkyl of more than six carbon atoms, aryl, or aralkyl.

(6) Thioalkanoic acid amide derivatives of Minagawa et al, published Japanese application Nol 106,484/74 having the formula:

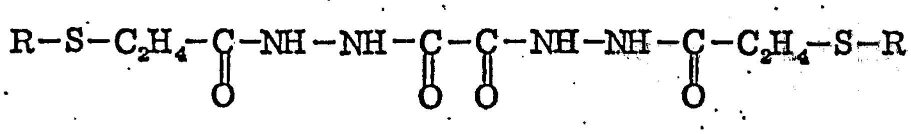

R is hydrocarbyl of one to twenty carbon atoms.

(7) Alkylene bis-thioalkanoic acid amides of U.S. patent No. 4,279,805 to Ohzeki et al, patented July 21, 1981, having the general formula:

$$R_1-S-R_2-\underset{\underset{O}{\|}}{C}-NH-R_3-NH-\underset{\underset{O}{\|}}{C}-R_2-S-R_1$$

wherein:

$R_1$ is alkyl having from one to about fifty carbon atoms;

$R_2$ is alkylene having from one to about three carbon atoms; and

$R_3$ is alkylene having from about two to about twelve carbon atoms.

β-Alkylthiopropionic acid esters having the general formula:

$$R-S-C_2H_4COOR-(R')_n$$

wherein:

R is alkyl of four to twenty carbon atoms;

n is a number from 1 to 6; and

R' is the residue of an alcohol having from one to six hydroxyl groups.

Pentaerythritol tetra dodecyl thio propionate is an example of this group.

A light stabilizer can be included. Particularly preferred are the 2,2,6,6-tetramethyl piperidine compounds which are known compounds that have in the molecule the group:

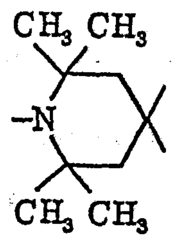

II

The 2,2,6,6-tetramethylpiperidine compounds are disclosed in the following illustrative patents:

Japanese patents Nos. 46-31733, 46-31734, 46-31735, 47-1628, 47-7380, 47-8539 and 48-12410; Japan kokai Nos. 46-5082, 47-590, 48-95986, 49-53570, 49-58085, 49-60337, 49-61236, 49-61238, 49-63738, 49-64634, 49-72332, 49-120492, 50-5435, 50-26779, 52-78876, 52-89677, 52-91875, 52-125175, 52-139071, 53-67749, 53-71082, 54-71185, 54-103877, 56-30985, 56-75488, 56-138189, 56-161387, 57-24393, 57-58681, 57-63359, 57-80453, 57-121034, 57-137358, 57-146755, 57-167316, 57-177022, 58-5319, 58-10568, 58-32642, 58-32864, 58-37025, 58-38720, 58-4703058-53931, 58-57444, 58-57445, 58-69879, 58-77862, 58-92660, 58-108238, 58-120646, 58-152053, 58-152881, 58-154739, 58-159460, 58-168634, 58-194862, 58-194931, 58-201777, 58-206594, 58-210094, 58-217554;

U.S. Patents Nos. 3,542,729, 3,640,928, 3,684,765, 3,705,126, 3,893,972, 3,925,376, 3,929,804, 3,940,401, 3,992,390, 3,899,464, 3,984,371, 3,971,795, 3,959,291, 3,993,655, 4,007,158, 4,038,280, 4,061,616, 4,086,204,

4,089,841, 4,096,114, 4,101,508, 4,102,858, 4,104,248, 4,104,251, 4,105,625, 4,107,139, 4,108,829, 4,110,334, 4,115,476, 4,116,927, 4,118,369, 4,128,608, 4,136,081, 4,140,673, 4,144,224, 4,148,784, 4,151,356, 4,154,722, 4,161,592, 4,162,246, 4,166,813; 4,173,599, 4,177,186, 4,185,007, 4,197,236, 4,198,334, 4,210,612, 4,219,465, 4,223,147, 4,234,728, 4,237,297, 4,238,388, 4,238,613, 4,276,401, 4,279,804, 4,288,593, 4,289,686, 4,293,466, 4,293,467, 4,293,468, 4,308,362, 4,309,546, 4,311,820, 4,312,804, 4,315,859, 4,316,025, 4,316,837, 4,317,911, 4,321,374, 4,322,531, 4,326,063, 4,331,586, 4,335,242, 4,336,183, 4,340,534, 4,348,524, 4,351,915, 4,356,279, 4,356,287, 4,356,307, 4,369,274, 4,369,321, 4,376,836, 4,378,443, 4,395,508, 4,400,513, 4,404,301, 4,405,735, 4,408,051, 4,412,021, 4,413,075, 4,413,076, 4,413,093 and 4,413,096.

Particularly preferred classes of 2,2,6,6-tetramethyl piperidyl compounds have the formulae III, IV and V:

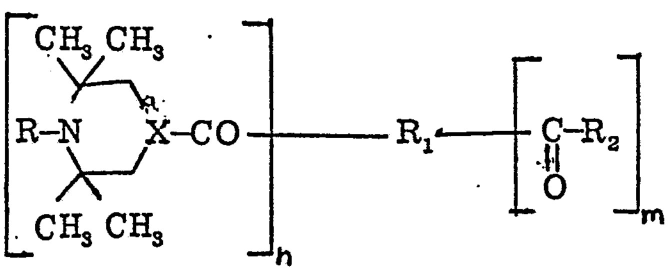

III

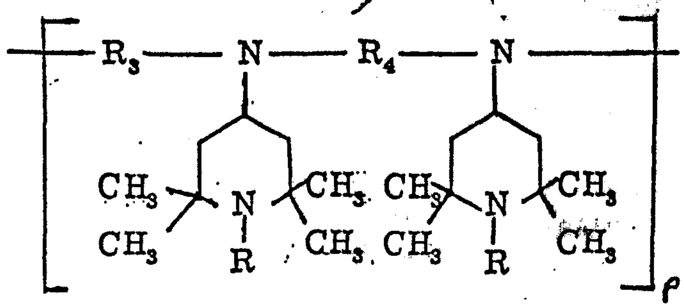

IV

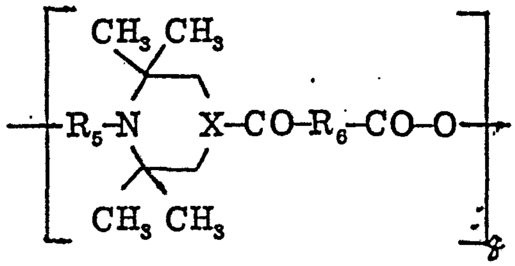

V

wherein;

R is selected from the group consisting of hydrogen; oxyl; alkyl having from one to about eighteen carbon atoms; and acyl having from one to about eighteen carbon atoms;

X is >CH–Y– or 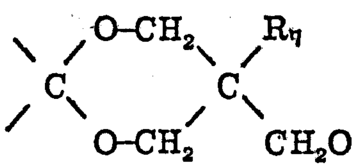 ;

Y is –O– or –N($R_8$)–;

$R_7$ is alkyl having from one to about five carbon atoms;

$R_8$ is hydrogen or alkyl having from one to about eighteen carbon atoms;

$R_1$ is a residue of mono- or poly-carboxylic acid;

n is a number from 1 to about 6;

m is a number from 0 to about 5; and

n + m is from 1 to about 6;

$R_2$ is –O–$R_8$ or –N($R_8$)$R_9$ ;

$R_9$ is alkyl having from one to about eighteen carbon atoms; and

$R_8$ and $R_9$ may be taken together to form alkylene or oxadialkylene;

$R_3$ is alkylene or 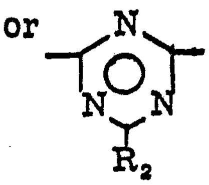

$R_4$ is alkylene;

$R_5$ is alkylene;

$R_6$ is alkylene or arylene;

p is a number from one to about twenty; and

q is a number from about 2 to about 20; p and q represent the number of units in the polymer molecule of IV and V.

Exemplary 2, 2, 6, 6-tetramethyl piperidine light stabilizers which can be employed together with the pentaerythritol bisphosphite include:

1. 4-Benzoyloxy-2, 2, 6, 6-tetramethylpiperidine

2. 1-(3, 5-Di-t-butyl-4-hydroxyphenylpropionyloxyethyl)-4-(3, 5-di-t-butyl-4-hydroxyphenylpropionyloxy)-2, 2, 6, 6-tetramethylpiperidine

3. 4-(β-3', 5'-Di-t-butyl-4-hydroxyphenylpropionyloxy)-2, 2, 6, 6-tetramethylpiperidine

4. Bis(2, 2, 6, 6-tetramethyl-4-piperidyl) sebacate

5. Bis(1, 2, 2, 6, 6-pentamethyl-4-piperidyl) sebacate

6. Bis(1, 2, 2, 6, 6-pentamethyl-4-piperidyl)-2-butyl-2-(3, 5-di-t-butyl-4-hydroxybenzyl) malonate

7. Bis(1-acryloyl-2, 2, 6, 6-pentamethyl-4-piperidyl)-2-butyl-2-(3, 5-di-t-butyl-4-hydroxybenzyl) malonate

8. Bis(9-aza-8, 8, 10, 10-tetramethyl-3-ethyl-1, 5-dioxaspiro [5.5]-3-undecylmethyl) methyliminodiacetate

9. Bis(2, 2, 6, 6-tetramethyl-4-piperidyl-1-oxyl) sebacate

10. Tris(2, 2, 6, 6-tetramethyl-4-piperidyl) citrate

11. Tris(2, 2, 6, 6-tetramethyl-4-piperidyl) nitrilotriacetate

12. Tris(2, 2, 6, 6-tetramethyl-4-piperidyl) butanetricarboxylate

13. Tris(2, 2, 6, 6-tetramethyl-4-piperidyl) trimellitate

14. Tetra(2, 2, 6, 6-tetramethyl-4-piperidyl) pyromellitate

15. Tetra(2, 2, 6, 6-tetramethyl-4-piperidyl)-1, 3-bis(aminomethyl) cyclohexanetetraacetate

16. Tetra(2, 2, 6, 6-tetramethyl-4-piperidyl)-1, 2, 3, 4-butanetetracarboxylate

17. Tris(2, 2, 6, 6-tetramethyl-4-piperidyl)-mono(isotridecyl)-1, 2, 3, 4-butanetetracarboxylate

18. Tetra(1, 2, 2, 6, 6-pentamethyl-4-piperidyl)-1, 2, 3, 4-butanetetracarboxylate

19. Tris(1, 2, 2, 6, 6-pentamethyl-4-piperidyl)-mono(isotridecyl)-1, 2, 3, 4-butanetetracarboxylate

20. Bis(1, 2, 2, 6, 6-pentamethyl-4-piperidyl)-di(isotridecyl)-1, 2, 3, 4-butanetetracarboxylate

21. Bis(2, 2, 6, 6-tetramethyl-4-piperidyl)-di(isotridecyl)-1, 2, 3, 4-butanetetracarboxylate

22. Bis(2, 2, 6, 6-tetramethyl-4-piperidyl-1-oxyl)-di(isotridecyl)-1, 2, 3, 4-butanetetracarboxylate

23. Mono(1, 2, 2, 6, 6-pentamethyl-4-piperidyl)-monomethylsebacate

24. 3, 9-Bis(1, 1-dimethyl-2-(tris(2, 2, 6, 6-tetramethyl-4-piperidyl-oxycarbonyl) butylcarbonyloxy) ethyl)-2, 4, 8, 10-tetraoxaspiro [5.5] undecane

25. 3, 9-Bis(1, 1-dimethyl-2-(tris(1, 2, 2, 6, 6-pentamethyl-4-piperidyl-oxycarbonyl) butylcarbonyloxy) ethyl)-2, 4, 8, 10-tetraoxaspiro [5.5] undecane

26. 2, 4, 6-Tris(2, 2, 6, 6-tetramethyl-4-piperidyloxy)-s-triazine

27. 2-Dibutylamino-4, 6-bis(9-aza-8, 8, 10, 10-tetramethyl-3-ethyl-1, 5-dioxaspiro [5.5] -3-undecylmethoxy)-s-triazine

28. N, N'-Bis(4, 6-bis(9-aza-8, 8, 10, 10-tetramethyl-3-ethyl-1, 5-dioxaspiro [5.5] -3-undecylmethoxy)-s-triazine-2-yl)piperazine

29. 1, 5, 8, 12-Tetrakis(4, 6-bis(N-(2, 2, 6, 6-tetramethyl-4-piperidyl) butylamino)-1, 3, 5-triazine-2-yl)-1, 5, 8, 12-tetraazadodecane

30. Bis(9-aza-8, 8, 10, 10-tetramethyl-3-ethyl-1, 5-dioxaspiro [5.5] -3-undecylmethyl) carbonate

31. Bis(9-aza-8, 8, 10, 10-tetramethyl-3-ethyl-1, 5-dioxaspiro [5.5] -3-undecylmethyl)-hydrogenatedbisphenol-A-dicarbonate

32. Bis(2, 2, 6, 6-tetramethyl-4-piperidyl)-pentaerythritol-diphosphite

33. Bis(9-aza-8, 8, 10, 10-tetramethyl-3-ethyl-1, 5-dioxaspiro [5.5] -3-undecylmethyl)-pentaerythritol-diphosphite

34. Tetra(2, 2, 6, 6-tetramethyl-4-piperidyl)-bisphenol-A-diphosphite

35. 3, 5-Di-t-butyl-4-hydroxybenzyl-bis(2, 2, 6, 6-tetramethyl-4-piperidyl) phosphonate

36. Condensate of 1-(2-hydroxyethyl)-2, 2, 6, 6-tetramethyl-4-piperidinol with dimethylsuccinate

37. Condensate of 2-t-octylamino-4, 6-dichloro-s-triazine with N, N'-bis-(2, 2, 6, 6-tetramethyl-4-piperidyl) hexamethylenediamine

38. Condensate of N, N'-bis(2, 2, 6, 6-tetramethyl-4-piperidyl) with hexamethylenediamine/dibromoethane

39. Bis(9-aza-8, 8, 10, 10-tetramethyl-3-ethyl-1, 5-dioxaspiro [5.5] -3-undecylmethyl) ether

40. 3-Glycidyl-8-methyl-7, 7, 9, 9-tetramethyl-1, 3, 8-triazaspiro [4.5] decane-2, 4-dione

41. 3-Dodecyl-8-acetyl-7, 7, 9, 9-tetramethyl-1, 3, 8-triazaspiro [4.5] decane-2, 4-dione

42. 3-Octyl-7, 7, 9, 9-tetramethyl-1, 3, 8-triazaspiro [4.5] decane-2, 4-dione

43. 2, 2, 4, 4-Tetramethyl-7-oxa-3, 20-diazadispiro [5.1.11.2] heneicosane-21-one

44. Condensate of 1, 6-bis(2, 2, 6, 6-tetramethyl-4-piperidylamino)-hexane with dibromoethane

45. N-(2, 2, 6, 6-tetramethyl-4-piperidyl) dodecyl-succinimide

Other conventional light stabilizers can be employed, such as hydroxybenzophenones such as 2-hydroxy-4-methoxybenzophenone, 2-hydroxy-4-n-octoxy benzophenone, 2,4-dihydroxybenzophenone, benzotriazoles, such as 2(2-hydroxy-5-methylphenyl) benzotriazoles, 2(2-hydroxy-3-t-butyl-5-methylphenyl)-5-chlorobenzotriazole, 2(2-hydroxy-3-5-di-t-butylphenyl) 5-chlorobenzotriazole, 2(2-hydroxy-3,5-di-t-amylphenyl) benzotriazole, benzoates such as phenylsalicylate, 2,4-di-t-butylphenyl -3,5-di-t-butyl-4-hydroxy phenylbenzoate, nickel compounds such as nickel-2,2'-thiobis(4-t-octyl-phenolate), nickel-monoethyl(3,5-di-t-butyl-4-hydroxybenzyl) phosphonate, substituted acrylonitriles such as methyl-$\alpha$-cyano-$\beta$-methyl-$\beta$-(p-methoxy phenyl)acrylate and oxalic anilides such as N-2-ethyl phenyl-N'-2-ethoxy-5-t-butyl phenyl oxalic diamide, N-2-ethyl phenyl-N'-2-ethoxy phenyl oxalic diamide.

A sufficient amount of the pentaerythritol bisphosphite is used to improve the resistance of the polyolefin to deterioration in physical properties when exposed to $\gamma$-radiation. Very small amounts are usually adeq uate. Amounts within the range from about 0.001 to about 10 parts by weight phosphite per 100 parts by weight polyolefin is satisfactory. Preferably, from 0.01 to 5 parts by weight is employed, for optimum stabilization.

The amount of phenolic antioxidant is within the range from 0.001 to about 5 parts by weight, and preferably 0.01 to 3 parts by weight, per 100 parts by weight of polyolefin resin.

The amount of thioether antioxidant is within the range from 0.001 to about 5 parts by weight, and preferably 0.01 to 3 parts by weight, per 100 parts by weight of polyolefin resin.

The amount of light stabilizer is within the range from 0.001 to about 5 parts by weight, and preferably 0.01 to 3 parts by weight, per 100 parts by weight of polyolefin resin.

The stabilizer is incorporated in the polymer in suitable mixing equipment, such as a mill or a Banbury mixer. If the polymer has a melt viscosity which is too high for the desired use, the polymer can be worked until its melt viscosity has been reduced to the desired range before addition of the stabilizer. Mixing is continued until the mixture is substantially uniform. The resulting composition is then removed from the mixing equipment and brought to the size and shape desired for marketing or use.

The stabilized polymer can be worked into the desired shape, such as by milling, calendering, extruding or injection molding or fiber-forming. In such operations, it will be found to have a considerably improved resistance to reduction in melt viscosity during the heating, as well as a better resistance to discoloration and embrittlement on ageing and heating.

The following Examples represent preferred embodiments of the invention:

Examples 1 and 2

The following polyolefin resin compositions were prepared, with organic phosphites of the prior art and of the invention:

| Ingredients | Parts by Weight |
| --- | --- |
| Propylene-ethylene copolymer (ethylene content: 2.5 wt. %) | 100 |
| Calcium stearate | 0.1 |
| Tetrakis(methylene-3-(3,5-di-tert-butyl-4-hydroxyphenyl) propionate) methane | 0.05 |
| Organic phosphite as shown in Table I | 0.2 |

The mixtures were pelletized by an extruder at 210°C, and the pellets injection-molded at 280°C to form test pieces 40 x 40 x 1 mm. Then, the test pieces were exposed to $\gamma$-rays of 2.5 megarads using a cobalt-60 radiation source.

The color difference (Hunter color difference, Lad) of the test pieces before and after the $\gamma$-ray irradiation was determined. Color difference values smaller than 1.5 imply practically no observable discoloration. A color difference value of 1.5 to 3.0 means a slight discoloration, while any color difference values in excess of 3.0 signify a large degree of discoloration.

The test pieces after irradiation were heated in an oven at 135°C to determine heat-resistance. The time in days to failure, i.e. the first discernible deteriation, such as cracking, discoloration, or embrittlement is noted as heat stability in Table I.

The results are shown in Table I:

Table I

| Example No. | Organic phosphite | Color Difference | Heat Stability |
|---|---|---|---|
| Control | Bis(2, 4-di-t-butylphenyl) pentaerythritol diphosphite | 3.4 | 8 days |
| Example 1 | Bis(2, 6-di-t-butyl-4-methylphenyl) pentaerythritol diphosphite | 1.7 | 22 days |
| Example 2 | Bis(2, 6-di-t-butyl-4-ethylphenyl) pentaerythritol diphosphite | 2.0 | 20 days |

The results from Examples 1 and 2 show that the composition of the present invention is scarcely discolored after exposure to $\gamma$-rays, and its heat stability after the exposure is also very excellent, as compared to the Control.

## Examples 3 to 11

The following polypropylene resin compositions were prepared, with organic phosphites of the invention:

| Ingredients | Parts by Weight |
|---|---|
| Polypropylene-homopolymer | 100 |
| Calcium stearate | 0.1 |
| Benzylidene sorbitol | 0.2 |
| Antioxidant as shown in Table II | As shown in Table II |
| Organic phosphite as shown in Table I | As shown in Table II |

The mixtures were pelletized by an extruder at 210°C, and the pellets injection-molded at 280°C to form test pieces 40 x 40 x 1 mm. Then, the test pieces were exposed to $\gamma$-rays of 3.5 megarads using a cobalt-60 radiation source.

The color difference (Hunter color difference, Lad) of the test pieces before and after the $\gamma$-ray irradiation was determined. Color difference values smaller than 1.5 imply practically no observable discoloration. A color difference value of 1.5 to 3.0 means a slight discoloration, while any color difference values in excess of 3.0 signify a large degree of discoloration.

The test pieces after irradiation were heated in an oven at 125°C to determine heat resistance. The time in days to failure, i.e. the first discernible deteriation, such as cracking, discoloration, or embrittlement is noted as heat stability in Table II.

The results are shown in Table II:

Table II

| Additive | Amount of Additive | | | | | | | | | | | |
|---|---|---|---|---|---|---|---|---|---|---|---|---|
| | Example Nos. | | | | | | | | | Controls | | |
| | 3 | 4 | 5 | 6 | 7 | 8 | 9 | 10 | 11 | 1 | 2 | 3 |
| Bis(2, 6-di-t-butyl-4-methylphenyl) pentaerythritol diphosphite | 0.1 | 0.1 | 0.1 | 0.1 | 0.1 | 0.1 | 0.1 | 0.1 | 0.1 | | | |
| Bis(2, 4-di-t-butylphenyl) pentaerythritol diphosphite | | | | | | | | | | 0.1 | 0.1 | 0.1 |
| Tetrakis(methylene-3-(3, 5-di-t-butyl-4-hydroxyphenyl) propionate) methane | 0.05 | 0.05 | 0.05 | 0.05 | 0.05 | 0.05 | 0.05 | | | 0.05 | 0.05 | 0.05 |
| 1, 3, 5-Tris(3, 5-di-t-butyl-4-hydroxybenzyl) isocyanurate | | | | | | | | 0.05 | 0.05 | | | |
| Bis(2, 2, 6, 6-tetramethyl-4-piperidyl) sebacate | | 0.1 | | | | | | | | | 0.1 | |
| Tetrakis(2, 2, 6, 6-tetramethyl-4-piperidyl) butanetetracarboxylate | | | 0.1 | | | | | 0.1 | | | | 0.1 |
| Bis(2, 2, 6, 6-tetramethyl-4-piperidyl)-di(tridecyl) butane-tetracarboxylate | | | | 0.1 | | | | | 0.1 | | | |
| 3, 9-Bis(1, 1-dimethyl-2-(tris(1, 2, 2, 6, 6-pentamethyl-4-piperidyloxycarbonyl)butylcarbonyl-oxy)ethyl)-2, 4, 8, 10-tetraoxaspiro [5. 5] undecane | | | | | 0.1 | | | | | | | |

Table II (continued)

| Additive | Amount of Additive | | | | | | | | | | | |
|---|---|---|---|---|---|---|---|---|---|---|---|---|
| | Example Nos. | | | | | | | | | Controls | | |
| | 3 | 4 | 5 | 6 | 7 | 8 | 9 | 10 | 11 | 1 | 2 | 3 |
| Condensate of 2-t-octylamino-4, 6-dichlorotriazine with 1, 6-bis (2, 2, 6, 6-tetramethyl-4-piperidylamino) hexane | | | | | | 0.1 | | | | | | |
| Condensate of 1, 6-bis(2, 2, 6, 6-tetramethyl-4-piperidylamino) hexane with dibromoethane | | | | | | | 0.1 | | | | | |
| Color Difference | 1.4 | 1.2 | 0.9 | 1.0 | 0.9 | 1.2 | 1.1 | 1.0 | 1.0 | 2.0 | 1.7 | 1.6 |
| Heat Stability Days | 13 | 28 | 34 | 32 | 32 | 30 | 30 | 33 | 32 | 7 | 19 | 22 |

The results for Examples 3 to 11, shown in Table II, demonstrate that the composition of the present invention is scarcely discolored after exposure to $\gamma$-rays, and its heat stability after the exposure is also very excellent.

CLAIMS:

1. A process for improving resistance to deterioration when exposed to sterilizing radiation of polyolefin resins which comprises combining with polyolefin resin an amount sufficient to improve resistance of the polyolefin resin to such deterioration of a phosphite having the formula:

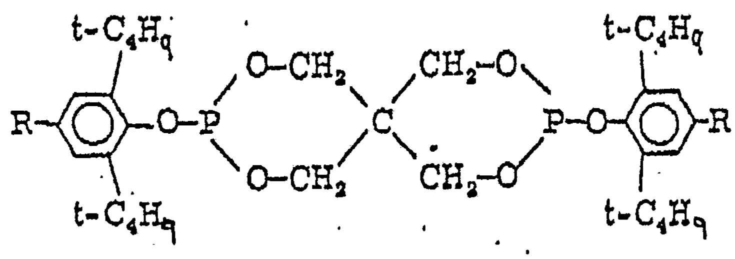

(I)

wherein R is alkyl having from one to about nine carbon atoms; and then exposing the polyolefin resin to sterilizing radiation.

2. A process according to claim 1, in which the amount of the phosphite is within the range from about 0.001 to about 10 parts by weight phosphite per 100 parts by weight polyolefin.

3. A process according to one or both of the claims 1 to 2 comprising in addition phenolic antioxidant in an amount within the range from 0.001 to about 5 parts by weight, per 100 parts by weight of polyolefin resin.

4. A process according to one or more of the claims 1 to 3 comprising in addition thioether antioxidant in an amount within the range from 0.001 to about 5 parts by weight, per 100 parts by weight of polyolefin resin.

5. A process according to one or more of the claims 1 to 4 comprising in addition light stabilizer in an amount within the range from 0.001 to about 5 parts by weight, per 100 parts by weight of polyolefin resin.

6. A process according to claim 5 in which the light stabilizer is a 2,2,6,6-tetramethyl piperidyl compound.

7. A process according to one or more of the claims 1 to 6 in which the phosphite is bis(2,6-di-t-butyl-4-methylphenyl) pentaerythritol diphosphite or bis(2,4-di-t-butylphenyl) pentaerythritol diphosphite.

8. A process according to one or more of the claims 1 to 7, in which the polypropylene is in the form of a shaped article of manufacture.

9. A process according to claim 8 in which the article is a medical instrument.